# EUROPEAN PATENT APPLICATION

(11) **EP 2 481 807 A2**
(43) Date of publication of application: **01.08.2012**
(21) Application number: 12157803.3
(22) Date of filing: 05.03.2008
(51) Int. Cl.: C12Q 1/00, C12Q 1/68, C12Q 1/70, C07H 21/00

(54) **In vivo HCV resistance to anti-viral inhibitors**

(30) Priority: 09.03.2007 US 906033 P
(62) Divisional of application: 08726439.6
(71) Applicant: Merck Sharp & Dohme Corporation, Rahway, NJ 07065 (US)
(72) Inventor: Ludmerer, Steven. W., Rahway New Jersey 07065-0907 (US); Graham, Donald J., Rahway New Jersey 07065-0907 (US); Olsen, David B., Rahway New Jersey 07065-0907 (US); Glaab, Warren E., Rahway New Jersey 07065-0907 (US)
(74) Representative: Horgan, James Michael Frederic

(57) **Abstract**

HCV mutations emerged in chimpanzees treated with a N5B polymerase inhibitor (Compound A) or a NS3 protease inhibitor (Compound B). Short term treatment with Compound A was followed by the initial emergence of an HCV with a S282T polymerase mutation following treatment. Short term treatment with Compound B selected for HCV with a R155K or D168T protease mutation.

## Description

### BACKGROUND OF THE INVENTION

The references cited in the present application are not admitted to be prior art to the claimed invention.

Exposure to hepatitis C virus (HCV) results in an overt acute disease in a small percentage of cases, while in most instances the virus establishes a chronic infection causing liver inflammation and slowly progresses into liver failure and cirrhosis. (Iwarson, FEMS Microbiol. Rev., 14:201-204, 1994.) Epidemiological surveys indicate HCV plays an important role in hepatocellular carcinoma pathogenesis. (Kew, FEMS Microbial. Rev., 14:211-220, 1994, Alter, Blood, 85:1681-1695, 1995.)

The HCV genome consists of a single strand RNA about 9.5 kb in length, encoding a precursor polyprotein about 3000 amino acids. (Choo et al., Science, 244:362-364, 1989, Choo et al., Science, 244:359-362, 1989, Takamizawa et al. J. Virol., 65:1105-1113, 1991.) The HCV polyprotein contains the viral proteins in the order: C-E1-E2-p7-NS2-NS3-NS4A-NS4B-NS5A-NS5B.

Individual viral proteins are produced by proteolysis of the HCV polyprotein. Host cell proteases release the putative structural proteins C, E1, E2, and p7, and create the N-terminus of NS2 at amino acid 810. (Mizushima et al., J. Virol., 68:2731-2734, 1994, Hijikata et al., Proc. Natl. Acad. Sci. USA., 90:10773-10777, 1993.)

The non-structural proteins NS3, NS4A, NS4B, NS5A and NS5B presumably form the virus replication machinery and are released from the polyprotein. A zinc-dependent protease associated with NS2 and the N-terminus of NS3 is responsible for cleavage between NS2 and NS3. (Grakoui et al., J. Virol., 67:1385-1395, 1993, Hijikata et al., Proc. Natl. Acad. Sci. USA, 90:10773-10777, 1993.)

A distinct serine protease located in the N-terminal domain of NS3 is responsible for proteolytic cleavages at the NS3/NS4A, NS4A/NS4B, NS4B/NS5A and NS5A/NS5B junctions. (Barthenschlager et al., J. Virol., 67:3835-3844, 1993, Grakoui et al., Proc. Natl. Acad. Sci. USA, 90: 10583-10587, 1993, Tomei et al., J. Virol. 67:4017-4026, 1993.) RNA stimulated NTPase and helicase activities are located in the C-terminal domain of NS3. NS4A provides a cofactor for NS3 protease activity. (Failla et al., J. Virol., 68:3753-3760, 1994, De Francesco et al., U.S. Patent No. 5,739,002.)

NS5B provides an RNA-dependent RNA polymerase. (De Francesco et al., U.S. Patent No. 6,383,768, Behrens et al., EMBO 15:12-22, 1996, Lohmann et al., Virology 249:108-118, 1998)

### SUMMARY OF THE INVENTION

HCV mutations emerged in chimpanzees treated with a NS5B polymerase inhibitor (Compound A) or a NS3 protease inhibitor (Compound B). Short term treatment with Compound A was followed by the initial emergence of an HCV with an amino acid substitution at position 282 of NS5B. Short term treatment with Compound B selected for HCV with an amino acid substitution at position 155 and/or 168 of NS3.

Thus, a first aspect of the present invention features a method of determining the existence of a HCV genotype resistant to a HCV protease inhibitor or HCV polymerase inhibitor. The method involves (1) removing HCV nucleic acid or polypeptide from a patient; and (2) determining whether the HCV present in the patient contains at least one amino acid substitution at position 282 of NS5B and/or any of positions 155 or 168 of NS3 that confers resistance to an anti-viral inhibitor.

In one embodiment, the amino acid substitution at position 282 of NS5B is threonine.

In another embodiment, the amino acid substitution at position 155 of NS3 is selected from the group consisting of lysine, methionine, serine, threonine or glutamine. In a preferred embodiment, the amino acid substitution at position 155 is lysine, methionine, serine or threonine. In a more preferred embodiment, the amino acid substitution at position 155 is lysine.

In another embodiment, the amino acid substitution at position 168 of NS3 is selected from the group consisting of alanine, glutamic acid, glycine, histidine, valine, tyrosine, asparagine, or threonine. In a preferred embodiment, the amino acid substitution at position 168 is glutamic acid, tyrosine or threonine. In a more preferred embodiment, the amino acid substitution at position 168 is threonine.

Preferably, the level of a particular mutation is quantified.

Removing HCV nucleic acid from a patient is achieved by obtaining a biological sample, such as plasma, containing HCV from the patient. Analysis can be performed on biological samples of varying degrees of purification. If desired, the nucleic acid or polypeptide is purified from most or all material originally present in the biological sample.

Reference to open-ended terms such as "comprises" allows for additional elements or steps. Occasionally phrases such as "one or more" are used with or without open-ended terms to highlight the possibility of additional elements or steps.

Unless explicitly stated reference to terms such as "a" or "an" is not limited to one. For example, "a cell" does not exclude "cells". Occasionally phrases such as one or more are used to highlight the possible presence of a plurality.

Other features and advantages of the present invention are apparent from the additional descriptions provided herein including the different examples. The provided examples illustrate different components and methodology useful in practicing the present invention. The examples do not limit the claimed invention. Based on the present disclosure the skilled artisan can identify and employ other components and methodology useful for practicing the present invention.

### BRIEF DESCRIPTION OF THE DRAWINGS

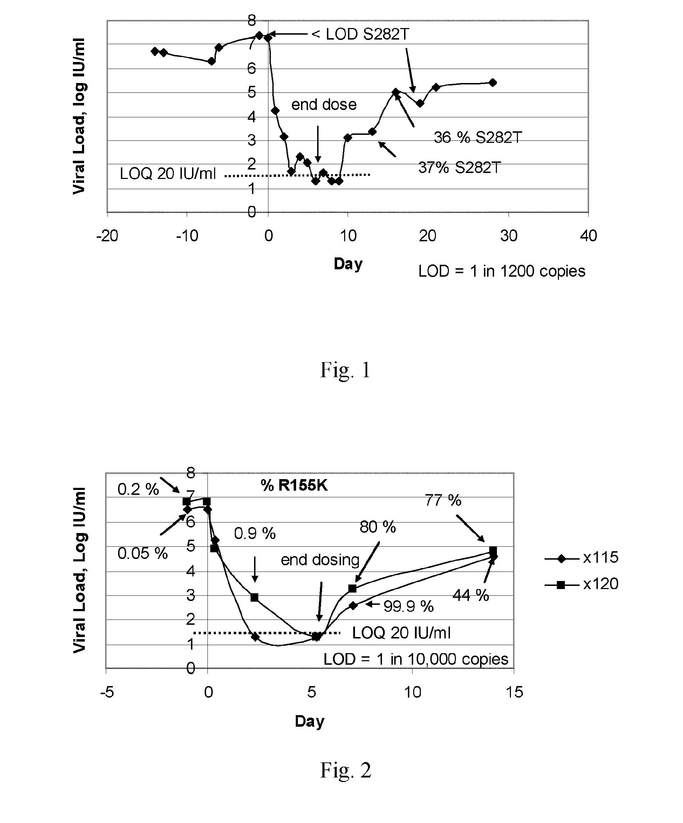

Figure 1 illustrates the effect of Compound A (a N5B polymerase inhibitor) on viral load and percentage of HCV containing the S282T mutation. LOQ is the lower limit of viral load quantitation, 20 IU/ml achieved in the commercially viral load assay used in these studies. The level of detection was 1 in 1,200 viral cDNA copies for the NS5B S282T. Compound A was administered for 7 days.

Abbreviations: LOQ= level of quantitation; < LOD= below the level of detection for the AUGER assay

Figure 2 illustrates the effect of Compound B (a NS3 protease inhibitor) on viral load and percentage of HCV containing the R155K mutation in two different Chimpanzees. LOQ is the lower limit of viral load quantitation, 20 IU/ml achieved in the commercially viral load assay used in these studies. The Chimpanzees were designated x115 and x120. LODwas approximately 1 in 10,000 viral cDNA copies for the NS3 R155K. Compound B was administered for 7 days.

Abbreviations: LOQ= level of quantitation; LOD= level of detection for the AUGER assay

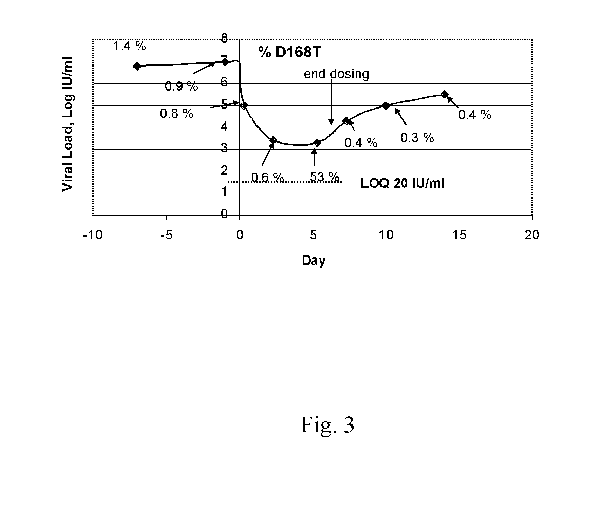

Figure 3 illustrates the effect of administering Compound B (a NS3 protease inhibitor) on viral load and percentage of HCV containing the D168T mutation in a single chimpanzee re-dosed with this compound. LOQ is the lower limit of viral load quantitation, 20 IU/ml achieved in the commercially viral load assay used in these studies. The Chimpanzee was designated x120. LOD was approximately 1 in 500 viral cDNA copies for the NS3 D168T. Compound B was administered for 7 days.

Abbreviations: LOQ= level of quantitation; LOD= level of detection for the AUGER assay

### DETAILED DESCRIPTION OF THE INVENTION

Chimpanzees infected with human HCV and treated with a N5B polymerase inhibitor (Compound A) or a NS3 protease inhibitor (Compound B) were found to give rise to HCV containing particular mutations. Dosing with either compound reduced the HCV level to below quantifiable levels. Subsequent to the short-term dosing, HCV levels relapsed. Emergence of HCV after Compound A treatment was initially characterized by a significant amount of HCV containing the S282T mutation, followed by a rapid reduction in HCV containing the S282T mutation (Figure 1). Emergence of HCV after Compound B treatment was characterized by an increased percentage of HCV containing the R155K mutation (Figure 2), which persisted for a longer period of time within the circulating viral populations. Re-dosing one of the chimpanzees with Compound B caused both the re-emergence of R155K, and an appearance of a D168T mutation (Figure 3). The identified mutations provide resistance to Compound A or Compound B.

The *in vivo* identification of the relevance of mutations at position 282 in NS5B and positions 155 and 168 of NS3 to treatment with Compound A and/or B can be used to help guide patient treatment. For example, treatment of patients containing these HCV mutations can be adjusted in or more of the following ways: increased dose, increased treatment duration, and use of a combination of different anti-HCV compounds.

In one embodiment, any amino acid at position 282 of NS5B other than wildtype indicates resistance. In a specific embodiment, an amino acid substitution at NS5B position 282 of threonine indicates resistance.

In another embodiment, any amino acid at position 155 of NS3 other than wildtype indicates resistance. In a specific embodiment, an amino acid substitution at position 155 of NS3 of lysine, methionine, serine, threonine or glutamine indicates resistance. In a more specific embodiment, an amino acid substitution at position 155 of lysine, methionine, serine or threonine indicates resistance. In a yet more specific embodiment, an amino acid substitution at position 155 of lysine indicates resistance.

In another embodiment, any amino acid at position 168 of NS3 other than wildtype indicates resistance. In a specific embodiment, an amino acid substitution at position 168 of NS3 of alanine, glutamic acid, glycine, histidine, valine, tyrosine, asparagine or threonine indicates resistance. In a more specific embodiment, an amino acid substitution at position 168 of NS3 of glutamic acid, tyrosine or threonine indicates resistance. In a yet more specific embodiment, an amino acid substitution at position 168 of NS3 of threonine indicates resistance.

### Compound A

Compound A is a nucleoside derivative that inhibits HCV NS5B polymerase activity. Compound A has the following structure:

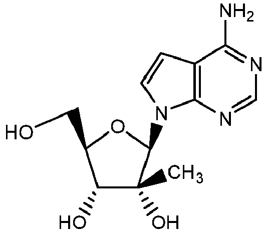

The production, formulation and use of Compound A is described in Carroll et al., U.S. Patent No. 7,105,499 (which is hereby incorporated by reference herein).

### Compound B

Compound B is a macrocyclic compound that inhibits HCV NS3 protease. Compound B has the following structure:

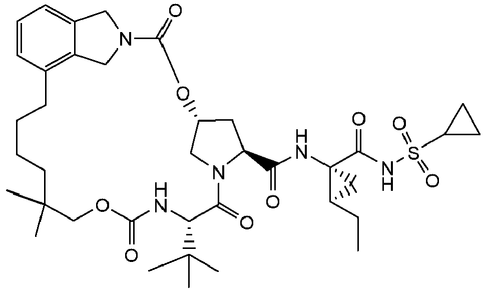

The production, formulation and use of Compound B is described in Holloway et al., Patent Publication No. US2007/0027071 (which is hereby incorporated by reference herein).

### Detection

HCV mutations can be detected based on the polypeptide sequence or encoding nucleic acid present in patient. Techniques for determining a polypeptide sequence are well known in the art. Examples of techniques for determining a polypeptide sequence include the use of amino acid stepwise chemical degradation with subsequent identification of released amino acids and the use of mass spectrometry. (Domon et al., Science 312:212-217, 2006.)

Techniques for detecting the presence of a particular nucleic acid mutation are well known in the art. Examples of such techniques include the use of nucleic acid hybridization probes, nucleic acid sequencing, MALDI-TOF spectrometry, and mismatch amplification TaqMan. (Ausubel, Current Protocols in Molecular Biology, John Wiley, 2005, Glaab et al., Mutat. Res. 430:1-12, 1999, Jurinke et al., Molecular Biotechnology 26:147-163, 2004.)

Allele-specific-Ultrasensitive Genotyping Assay for Enzyme Resistance (AUGER) is a preferred method to identify and quantify the presence of mutant HCV virus within an HCV-infected plasma sample resistant to HCV antiviral compounds. The method can be used to detect and monitor the presence or emergence of resistant variants.

As described in Examples below, AUGER can be performed by applying mismatch amplification TaqMan (Glaab et al., Mutat. Res. 430:1-12, 1999) to HCV-infected plasma samples to quantify viral resistance based on particular mutations. Allele discrimination is provided by the wild-type and mutant discrimination primers, which serve as one of the two PCR primers for a TaqMan-based assay. The last nucleotide of the primers encode either the wild-type or resistant mutant base, while the penultimate base is intentionally mismatched in both. The primers are designed to have a Tm between 56-60°C, ensuring a difference in cycle thresholds between mutant and wild-type templates. Data analysis can be conducted according to standard TaqMan methodology.

The present application demonstrates that the S282T polymerase mutation emerges in chimps infected with a HCV 3a genotype and treated for a period of time with a N5B polymerase inhibitor (Compound A). In different embodiments, the presence of threonine in position 282 from a patient is determined; the HCV is genotype 3a or 1a; nucleic acid is isolated from the patient and the presence HCV genotype 3a or 1a polymerase comprising threonine in position 282 is determined using a primer of SEQ ID NO: 11; and/or the method further employs the use of a primer of SEQ ID NO: 12 and a probe comprising SEQ ID NO: 13.

The S282T mutation is also present in HCV replicons. (Migliaccio et al., J. Biol. Chem. 278:49164-49170, 2004, Klumpp et al., The Journal of Biological Chemistry 281(7):3793-3799, 2006, Pogam et al., Virology 351:349-359, 2006, Olsen et al., Antimicrobial Agents and Chemotherapy 48:3944-3953, 2004.)

The R155K and D168T protease mutations were identified in chimps infected with a HCV 1a genotype and treated with Compound B (a NS3 protease inhibitor). In different embodiments, the presence of a lysine in position 155 or a threonine in position 168 of HCV protease from a patient is determined; the HCV is genotype is 1; the HCV is genotype 1a; nucleic acid is isolated from the patient and the presence of the HCV genotype 1a NS3 comprising lysine in position 155 is determined using a primer of SEQ ID NO: 2 or 5 and/or the method further employs a further primer of SEQ ID NO: 3 and a probe comprising SEQ ID NO: 6.

The R155K mutation is associated with resistance to Telaprevir. (Kieffer et al., 57th Annual Meeting of the American Association of the Study of Liver Disease, October 30, 2006, Boston MA.). The D168T mutation has not been previously described.

### Administration

Compounds can be formulated and administered to a patient using the guidance provided herein, Holloway et al., Patent Publication No. US2007/0027071, Carroll et al., US Patent No. 7,105,499, along with techniques well known in the art. The preferred route of administration ensures that an effective amount of compound reaches the target. Guidelines for pharmaceutical administration in general are provided in, for example, Remington's Pharmaceutical Sciences 18th Edition, Ed. Gennaro, Mack Publishing, 1990, and Modern Pharmaceutics 2nd Edition, Eds. Banker and Rhodes, Marcel Dekker, Inc., 1990, both of which are hereby incorporated by reference herein.

Compounds may be administered in the form of pharmaceutically acceptable salts. The term "pharmaceutically acceptable salt" refers to a salt suitable for administration to a patient. Suitable salts include acid addition salts which may, for example, be formed by mixing a solution of compound with a solution of a pharmaceutically acceptable acid such as hydrochloric acid, sulfuric acid, acetic acid, trifluoroacetic acid, or benzoic acid. For compounds carrying an acidic moiety, pharmaceutically acceptable salts thereof can include alkali metal salts (e.g., sodium or potassium salts), alkaline earth metal salts (e.g., calcium or magnesium salts), and salts formed with suitable organic ligands such as quaternary ammonium salts. Also, in the case of an acid (-COOH) or alcohol group being present, pharmaceutically acceptable esters can be employed to modify the solubility or hydrolysis characteristics of the compound.

Reference to "treating" includes treating a patient infected with HCV or treating a patient prophylactically. A "patient" refers to a mammal capable of being infected with HCV. A patient may or may not be infected with HCV. Examples of patients are humans and chimpanzees, preferably the patient is a human.

An effective amount of compound can be used for treatment of an active infection. For a patient infected with HCV, an effective amount is sufficient to achieve one or more of the following effects: reduce the ability of HCV to replicate, reduce HCV load, and increase viral clearance.

Compounds optionally in the form of a salt or a hydrate, can be administered by different routes including oral, nasal, by injection, transdermal, and transmucosally. Active ingredients to be administered orally as a suspension can be prepared according to techniques well known in the art of pharmaceutical formulation and may contain microcrystalline cellulose for imparting bulk, alginic acid or sodium alginate as a suspending agent, methylcellulose as a viscosity enhancer, and sweeteners/flavoring agents. As immediate release tablets, these compositions may contain microcrystalline cellulose, dicalcium phosphate, starch, magnesium stearate and lactose and/or other excipients, binders, extenders, disintegrants, diluents and lubricants.

When administered by nasal aerosol or inhalation, compositions can be prepared according to techniques well known in the art of pharmaceutical formulation. Such compositions may be prepared, for example, as solutions in saline, employing benzyl alcohol or other suitable preservatives, absorption promoters to enhance bioavailability, fluorocarbons, and/or other solubilizing or dispersing agents.

The compounds may also be administered in intravenous (both bolus and infusion), intraperitoneal, subcutaneous, topical with or without occlusion, or intramuscular form, all using forms well known to those of ordinary skill in the pharmaceutical arts. When administered by injection, the injectable solutions or suspensions may be formulated using suitable non-toxic, parenterally-acceptable diluents or solvents, such as Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

The compounds can be administered orally in a dosage range of 0.001 to 1000 mg/kg of mammal (e.g., human) body weight per day in a single dose or in divided doses. One dosage range is 0.01 to 500 mg/kg body weight per day orally in a single dose or in divided doses. Another dosage range is 0.1 to 100 mg/kg body weight per day orally in single or divided doses. For oral administration, the compositions can be provided in the form of tablets or capsules containing 1.0 to 500 milligrams of the active ingredient, particularly 1, 5, 10, 15, 20, 25, 50, 75, 100, 150, 200, 250, 300, 400, and 500 milligrams of the active ingredient for the symptomatic adjustment of the dosage to the patient to be treated. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the patient undergoing therapy.

### Adjusting Treatment

Treatment for patients determined to be infected with HCV containing a mutation in the NS5B polymerase and/or the NS3 protease that confers resistance to inhibitors at position can be adjusted to increase the effectiveness of treatment with a N5B polymerase inhibitor (e.g., Compound A) and/or a NS3 protease inhibitor (e.g., Compound B). Treatment adjustments include one or more of: increased dose, increased treatment duration, and use of a combination of different anti-HCV compounds.

In one embodiment, any amino acid at position 282 of NS5B other than wildtype indicates resistance. In a specific embodiment, an amino acid substitution at NS5B position 282 of threonine indicates resistance.

In another embodiment, any amino acid at position 155 of NS3 other than wildtype indicates resistance. In a specific embodiment, an amino acid substitution at position 155 of NS3 of lysine, methionine, serine, threonine or glutamine indicates resistance. In a more specific embodiment, an amino acid substitution at position 155 of lysine, methionine, serine or threonine indicates resistance. In a yet more specific embodiment, an amino acid substitution at position 155 of lysine indicates resistance.

In another embodiment, any amino acid at position 168 of NS3 other than wildtype indicates resistance. In a specific embodiment, an amino acid substitution at position 168 of NS3 of alanine, glutamic acid, glycine, histidine, valine, tyrosine, asparagine or threonine indicates resistance. In a more specific embodiment, an amino acid substitution at position 168 of NS3 of glutamic acid, tyrosine or threonine indicates resistance. In a yet more specific embodiment, an amino acid substitution at position 168 of NS3 of threonine indicates resistance.

The proportion of HCV in a patient with one or more mutations in NS5B and/or NS3 that confer resistance can be determined. Quantifying the level of mutant virus can aid in determination of treatment adjustment. In one embodiment, treatment is adjusted if at least 5% of the HCV in a patient sample has one or more mutations in NS5B and/or NS3 that confer resistance. In another embedment, treatment is adjusted if at least 10% of the HCV in a patient sample has one or more mutations in NS5B and/or NS3 that confer resistance. In another embedment, treatment is adjusted if at least 25% of the HCV in a patient sample has one or more mutations in NS5B and/or NS3 that confer resistance. In another embedment, treatment is adjusted if at least 50% of the HCV in a patient sample has one or more mutations in NS5B and/or NS3 that confer resistance. In another embedment, treatment is adjusted if at least 75% of the HCV in a patient sample has one or more mutations in NS5B and/or NS3 that confer resistance. In another embedment, treatment is adjusted if at least 90% of the HCV in a patient sample has one or more mutations in NS5B and/or NS3 that confer resistance. In another embedment, treatment is adjusted if at least 95% of the HCV in a patient sample has one or more mutations in NS5B and/or NS3 that confer resistance. In another embedment, treatment is adjusted if at least 99% of the HCV in a patient sample has one or more mutations in NS5B and/or NS3 that confer resistance.

Different combination treatments are possible. Combination treatment involving Compound A include those described in U.S. Patent No. 7,105,499. Combination treatment involving Compound B include those described in Patent Publication No. US20070027071. Other examples of compounds that can be used with Compound A or B are provided in Holloway et al., International Publication No. WO2007/016441 (*e.g*., compound III-131), Conte et al., International Publication No. WO2006/046030 (Example 12, *N'*-[(7R)-11-carboxy-14-cyclohexyl-7,8-dihydro-6*H*-indolo[1,2-e][1,5]benzoxazocin-7-yl]-*N*,*N*,*N*'-trimethylethane-1,2-diaminium bis(trifluoroacetate), Klumpp et al., The Journal of Biological Chemistry 281(7):3793-3799, 2006, Pogam et al., Virology 351:349-359, 2006, and Olsen et al., Antimicrobial Agents and Chemotherapy 48:3944-3953, 2004. In an embodiment, Compound A and Compound B are used together.

### Examples

Examples are provided below further illustrating different features of the present invention. The examples also illustrate useful methodology for practicing the invention. The examples do not limit the claimed invention.

### Example 1: R155K mutation

Two chimpanzees chronically infected with HCV genotype 1a from a human were orally dosed with Compound B (a NS3 protease inhibitor) for 7 days at 5 mg per kg b.i.d. Plasma samples were collected prior, during, and after the dosing regimen, with viral load determined by a commercial viral load assay (RT-TaqMan, level of quantitation 20 IU/ml, Cenetron Diagnostics, Austin, TX 78758). NS3/4a cDNA was generated from the plasma samples by RT-PCR and the cDNA sequenced directly; an R155K change (AGG to AAG) was determined by this population sequencing to be the major genetic change within the rebounding viral population which distinguished it from the pre-dose virus.

NS3/4a cDNA generated from viral RNA of day -1, day 0 (8 hours after administration of the initial dose), day 2 (chimpanzee x120 only), day 7, and day 14 (7 days post-dosing) evaluated by AUGER to quantify the level of R155K virus. 10⁶ copies of cDNA template were used per AUGER reaction.

Primer and probe sequences were designed using Primer Express software (Applied Biosystems). Primers were designed to have a Tm between 56-60°C.

### i) Primers for chimpanzee X115:

wild-type (R155R): 5'- CGCCGTGGGCCTATTT(C or G)G (SEQ ID NO: 1)
mutant (R155K): 5'- CGCCGTGGGCCTATTT(C or G)A (SEQ NO: 2)

The final G of the wild-type primer matches the 2^{nd} nucleotide of the arginine AGG codon, the final A of the mutant primer matches the 2^{nd} nucleotide of the lysine AAG codon, and the penultimate base is intentionally mis-matched for both primers. A reverse primer (5'-GGGATAAAGTCCACCGCCTTA, SEQ NO: 3) was employed for both the wild-type and mutant AUGER reactions.

### ii) Primers chimpanzee x120:

wild-type (R155R): 5' CACGCCGTAGGCATATTC(C or T)G (SEQ ID NO: 4)
mutant (R155K): 5' CACGCCGTAGGCATATTC(C or T)A (SEQ ID NO: 5)

The final G of the wild-type primer matches the 2^{nd} nucleotide of the arginine AGG codon, the final A of the mutant primer matches the 2^{nd} nucleotide of the lysine AAG codon, and the penultimate base is intentionally mis-matched for both primers. A reverse primer (SEQ ID NO: 3) was employed for both the wild-type and mutant AUGER reactions.

### (iii) Probe

The TaqMan probe was 6 fam- TGTGCACCCGTGGAG (SEQ ID NO: 6)-mgbnfq, where 6 fam is 6-carboxyfluorescein and mgbnfq is a minor groove binder non-fluorescent quencher (Taqman MGB probe, cat. # 4316034, Applied Biosystems, Foster City, CA). The TaqMan probe was used for both the x115 and x120 AUGER reactions.

For each plasma time point Ct determinations were made for both wild-type and mutant discriminating primers using 10⁶copies of NS3/4a cDNA template derived from each plasma sample. Determination of the percentage of the sample that was wild-type or R155K virus was made using standard TaqMan calculations of the Ct (cycle threshold) experimentally determined for both wild-type and R155K discriminating primers, using the assumption that the total viral population was comprised of wild-type and R155K virus. There was no experimental evidence for any other viral mutation at this amino acid position. The results are shown in Table 1.

**Table 1**

| AUGER Analysis : % R155K Virus | | |
|---|---|---|
| Time | Chimpanzee X115 | Chimpanzee X120 |
| -1 Days | 0.05 | 0.2 |
| 0 Days | 0.05 | 0.9 |
| 2 Days | ND | 98.8 |
| 7 Days | 99.3 | 80 |
| 14 Days | 44 | 77 |
| 33 Days | 14 | ND |
| 40 Days | ND | 28 |
| 8 Months | ND | 9 |

| | | |
|---|---|---|
| "ND" indicates no data. | | |

The viral load determination for the study, with the percentage of R155K virus shown beside the respective time points is illustrated in Figure 2. For this set of experiments the sensitivity of R155K detection was approximately 0.01 %, or 1 copy per 10,000 templates. This sensitivity was determined by the difference in Ct for the R155K discriminating primer against control mutant versus wild-type templates generated from cloned genes.

Administration of the protease inhibitor resulted in rapid viral load suppression to below the limit of quantitation (BLQ). After Compound B (a NS3 protease inhibitor) treatment, early rebound viral populations were enhanced for resistant virus (R155K), but there was no evidence for antiviral failure during dosing. Careful analysis showed a very small population (<0.1%) of this resistant virus in pretreatment samples. Virus resistant to the protease inhibitor (R155K) which emerged following cessation of dosing diminished gradually and persisted as a minor species for several months.

### Example 2: D168T mutation

Several months after this initial study chimpanzee x120 was re-dosed orally with Compound B (a NS3 protease inhibitor) for 7 days at 10 mg per kg q.d. Plasma samples were collected prior, during, and after the dosing regimen, with viral load determined by a commercial viral load assay (RT-TaqMan, level of quantitation 20 IU/ml, Cenetron Diagnostics, Austin, TX 78758). NS3/4a cDNA was generated from the plasma samples by RT-PCR and the cDNA sequenced directly. A homogenous R155K change (AGG to AAG) was determined by this population sequencing to be the major genetic change at day 2 and within the rebounding viral population. Unlike the homogenous R155K sequence at day 2, the day 5 sample was heterogenous R155K. In addition, it was also heterogenous at both the first (G/A) and second positions (A/C) of codon 168, potentially encoding D (wt), A, N, or T. Sequencing 19 molecular clones demonstrated that they encoded either R155K and wild-type at 168, or wild-type at 155 and D168T (one clone was wild-type at both positions).

NS3/4a cDNA generated from viral RNA of day -1, day 0 (8 hours after administration of the initial dose), day 2, day 5, day 7, day 10, and day 14 (7 days post-dosing) evaluated by AUGER to quantify the level of R155K virus. 10⁶ copies of cDNA template were used per AUGER reaction

Primer and probe sequences were designed using Primer Express software (Applied Biosystems). Primers were designed to have a Tm between 56-60°C.

### ii) Primers for D168T mutation:

wild-type (D168D): 5' AGTGGCTAAGGCGGTGGA (SEQ ID NO: 7)
mutant (D168T): 5' AGTGGCTAAGGCGGTGAC (SEQ ID NO: 8)

Because the D and T codons differ at both the first and second nucleotides, the discriminating primers were directly matched to the D and T codons respectively. A reverse primer (5'-TTGGACATGTAAGCACCAAAGC, SEQ ID NO: 9) was employed for both the wild-type and mutant AUGER reactions. Detection was monitored using SYBR Green.

For each plasma time point Ct determinations were made for both wild-type and mutant discriminating primers using 10⁶ copies of NS3/4a cDNA template derived from each plasma sample. Determination of the percentage of the sample that was wild-type or D168T virus was made using standard TaqMan calculations of the Ct (cycle threshold) experimentally determined for both wild-type and D 168T discriminating primers, using the assumption that the total viral population was comprised of wild-type and D168T virus. There was no experimental evidence for any other viral mutation at this amino acid position. Determination of the percentage of these samples that was wild-type or R155K was performed as described in Example 1, with the exception that detection was monitored using SYBR Green rather than the previously described probe (SEQ ID NO: 6). The results are shown in Table 2.

**Table 2**

| AUGER Analysis : Chimp x120 | | |
|---|---|---|
| Time | % R155K | % D168T |
| -7 Days | 0.2 | 1.4 |
| -1 Days | 0.1 | 0.9 |
| 0 Days | 0.1 | 0.8 |
| 2 Days | 98 | 0.6 |
| 5 Days | 48 | 53 |
| 7 Days | 89 | 0.4 |
| 10 Days | 61 | 0.3 |
| 14 Days | 36 | 0.4 |

The viral load determination for the study, with the percentage of R155K or D168T virus shown beside the respective time points are illustrated in Figure 2. For this set of experiments the sensitivity of R155K detection was approximately 0.01 %, or 1 copy per 10,000 templates, while the sensitivity for D168T detection was approximately 0.2 %, or 1 copy per 500 templates. This sensitivity was determined by the difference in Ct for the R155K or D168T discriminating primer against control mutant versus wild-type templates generated from cloned genes.

### Example 3: S282T Mutation for genotype 3a

A chimpanzee infected with a HCV genotype 3a from a human was dosed with Compound A (a N5B polymerase inhibitor) for 7 days at 2 mg per kg IV. Plasma samples were collected prior, during, and after the dosing regimen, with viral load determined by a commercial viral load assay (RT-TaqMan, level of quantitation 20 IU/ml, Cenetron Diagnostics, Austin, TX 78758). NS5B cDNA was generated from select plasma samples by RT-PCR and the cDNA sequenced directly; an S282T change (AGT to ACT) was determined by this population sequencing to be the major genetic change within the rebounding viral population.

NS5B cDNA generated from viral RNA of day -1, day 13, dayl6, and day 19 (13 days post-dosing) evaluated by AUGER to quantify the level of S282T virus. 10⁶ copies of cDNA template were used per AUGER reaction

Primer and probe sequences were designed using Primer Express software (Applied Biosystems). Primers were designed to have a Tm between 56-60°C.
wild-type primer rev: 5'- AGCTGGTAGGCAAAACTCCTC (SEQ ID NO: 10)
S282T mutant primer rev: 5' AGCTGGTAGGCAAAACTCCTG (SEQ ID NO: 11)
Forward PCR primer: GGAGGCCAGGAAAGTGATCT (SEQ ID NO: 12)
Probe: 6-fam-CCTCACGGAGCGGCT (SEQ ID NO: 13)-mgbnfq

For each plasma time point Ct determinations were made for both wild-type and mutant discriminating primers using 10⁶ copies of NS5B cDNA template derived from the selected plasma samples. Determination of the percentage of the sample that was wild-type or S282T virus was made using standard TaqMan calculations of the Ct (cycle threshold) experimentally determined for both wild-type and S282T discriminating primers, using the assumption that the total viral population was comprised of wild-type and S282T virus. There was no experimental evidence for any other viral mutation at this amino acid position.

The results are shown in Figure 1. For this set of experiments the sensitivity of S282T detection was approximately 1 copy per 1200 templates. This sensitivity was determined by the difference in Ct for the S282T discriminating primer against control mutant versus wild-type templates generated from cloned genes.

Administration of the polymerase inhibitor resulted in rapid viral load suppression to below the limit of quantitation (BLQ). Viral load rebounded post-dosing, although remaining BLQ for a few days following cessation of this short-term dosing. Virus resistant to the polymerase inhibitor (S282T) emerged in the early rebound populations sampled 7 and 10 days upon cessation of dosing (days 13 and 16 of the study), but disappeared from the circulating population 13 days post-dosing (day 19 of the study), suggesting debilitated replication.

### Example 4: S282T Mutation

A chimpanzee infected with a HCV genotype 1a from a human was dosed with Compound A (a N5B polymerase inhibitor) for 7 days at 2 mg per kg IV. Plasma samples were collected prior, during, and after the dosing regimen, with viral load determined by a commercial viral load assay (RT-TaqMan, level of quantitation 20 IU/ml, Cenetron Diagnostics, Austin, TX 78758). NS5B cDNA was generated from select plasma samples by RT-PCR and the cDNA sequenced directly; no evidence for genetic heterogeneity at Ser282 was detected by this population sequencing.

NS5B cDNA generated from viral RNA of day -1, day 1, day 9, and day 13 (6 days post-dosing) evaluated by AUGER to quantify the level of S282T virus. 10⁶ copies of cDNA template were used per AUGER reaction

Primer and probe sequences were designed using Primer Express software (Applied Biosystems). Primers were designed to have a Tm between 56-60°C.
wild-type primer reverse: 5'- CTAGTTGTCAGCACGCCAC (SEQ ID NO: 14)
S282T mutant primer reverse: 5' CTAGTTGTCAGCACGCCAG (SEQ ID NO: 15)
forward PCR primer: CCGCTGTTTTGACTCCACAGT (SEQ ID NO: 16)
Probe: 6-fam-ACTGAGAACGACATCCGT (SEQ ID NO: 17)-mgbnfq

For each plasma time point Ct determinations were made for both wild-type and mutant discriminating primers using 10⁶ copies of NS5B cDNA template derived from the selected plasma samples. Determination of the percentage of the sample that was wild-type or S282T virus was made using standard TaqMan calculations of the Ct (cycle threshold) experimentally determined for both wild-type and S282T discriminating primers, using the assumption that the total viral population was comprised of wild-type and S282T virus. There was no experimental evidence for any other viral mutation at this amino acid position.

There was no evidence by AUGER for S282T virus from any of the samples analyzed. S282T virus for any of the samples was below the limit of detection, which for this set of experiments was < 1 in 100,000 templates. This sensitivity was determined by the difference in Ct for the S282T discriminating primer against control mutant versus wild-type templates generated from cloned genes. In this chimpanzee S282T virus either does not exist, or is in such minute quantities that was undetectable.

Other embodiments are within the following claims. While several embodiments have been shown and described, various modifications may be made without departing from the spirit and scope of the present invention.

## Claims

1. A method of determining the existence of a hepatitis C virus (HCV) genotype resistant to a HCV protease inhibitor or HCV polymerase inhibitor comprising the steps of:
a) removing HCV nucleic acid or polypeptide from a patient; and
b) determining whether HCV present in said patient comprises at least one amino acid substitution in:
i) position 155 of NS3;
ii) position 282 of NS5B; and
iii) position 168 of NS3,
wherein one or more amino acid substitutions indicates resistance.

2. The method of claim 1, wherein the at least one amino acid substitution is selected from the group consisiting of:
a) a threonine at position 282 of NS5B;
b) a lysine, methionine, serine, threonine or glutamine at position 155 of NS3; and
c) an alanine, glutamic acid, glycine, histidine, valine, tyrosine, asparagine or threonine at position 168 of NS3.

3. The method of claim 2, wherein the at least one amino acid substitution is selected from the group consisiting of:
a) a lysine at position 155 of NS3; and
b) a threonine at position 168 of NS3.

4. The method of any of claims 1-3, wherein said NS3 is from an HCV genotype 1.

5. The method of claim 4, wherein said HCV genotype is 1a.

6. The method of any of claims 1-3, wherein said NS5B is from an HCV genotype 3 a or an HCV genotype 1a.

7. The method of any of claims 1-6, wherein said method further comprises quantifying the level of said HCV that has at least one amino acid substitution.

8. The method of any of claims 1-7, wherein nucleic acid is isolated from said patient and the presence of a lysine at position 155 of NS3 is determined using a primer of SEQ ID NO: 2 or 5.

9. The method of claim 8, wherein said method further comprises the use of a reverse primer of SEQ ID NO: 3 and a probe comprising SEQ ID NO: 6.

10. The method of any of claims 1-7, wherein nucleic acid is isolated from said patient and the presence of a threonine at position 168 of NS3 is determined using a primer of SEQ ID NO: 8.

11. The method of claim 10, wherein said method further comprises the use of a reverse primer of SEQ ID NO: 9.

12. The method of any of claims 1-7, wherein nucleic acid is isolated from said patient and the presence of a threonine at position 282 of NS5B is determined using a primer of SEQ ID NO: 11.

13. The method of claim 12, wherein said method further comprises the use of a forward primer of SEQ ID NO: 12 and a probe comprising SEQ ID NO: 13.

14. A nucleic acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16 and SEQ ID NO:17.
